# EUROPEAN PATENT SPECIFICATION

(11) **EP 3 356 822 B1**
(45) Date of publication and mention of the grant of the patent: **27.10.2021**
(21) Application number: 16856992.9
(22) Date of filing: 30.09.2016
(51) Int. Cl.: G01N 33/542, G01N 33/566, G01N 33/53, G01N 33/543

(54) **ANALYTE DETECTION WITH MULTIPLE SUBSTRATES**
NACHWEIS VON ANALYTEN MIT MEHREREN SUBSTRATEN
DÉTECTION D'ANALYTE AVEC DE MULTIPLES SUBSTRATS

(30) Priority: 02.10.2015 US 201562236676 P; 05.10.2015 US 201562237522 P
(43) Date of publication of application: 08.08.2018
(73) Proprietor: TGR Biosciences Pty Ltd., Thebarton, South Australia 5031 (AU)
(72) Inventor: SHEEHAN, Antony, James, Seaview Downs, South Australia 5049 (AU); CROUCH, Michael, Francis, Thebarton, South Australia 5031 (AU)
(74) Representative: Boult Wade Tennant LLP
(86) International application number: PCT/IB2016/001981
(87) International publication number: WO 2017/068431

(56) References cited:
- WO-A1-2010/095033
- WO-A1-2012/129611
- US-A1- 2003 232 394
- Ilene Schneider: "PerkinElmer Launches Innovative Multiplexing System AlphaPlex(TM) Reagent Technology Provides Life Sciences Researchers with More Data in Less Time", , 2 September 2014 (2014-09-02), XP055591765, Retrieved from the Internet: URL:http://ir.perkinelmer.com/static-files /1d369aa8-6206-44d3-bf8e-9e7efc4cb560 [retrieved on 2019-05-24]
- Perkin Elmer: "AlphaPlex Assay Development Guide", , 14 December 2015 (2015-12-14), XP055591764, Retrieved from the Internet: URL:https://www.perkinelmer.co.uk/lab-solu tions/resources/docs/GDE_AlphaPLEX_Assay_D evelopment_Guide_2015_Final.pdf [retrieved on 2019-05-24]
- "TAKING PROTEIN PHOSPHORYLATION MEASUREMENT ONE STEP FURTHER", PerkinElmer brochure, August 2014 (2014-08), XP009510111, Retrieved from the Internet: URL:https://www.perkinelmer.com/lab- solutions/resources/docs/BRO_AlphaLISA_Sur eFire_Uttra.pdf
- EGLEN, RICHARD M. et al.: "The use of AlphaScreen technology in HTS: current status.", Current chemical genomics, vol. 1, no. 1, 2008, pages 2-10, XP008176468,
- "CLONING AND EXPRESSION", Sigma, 18 March 2015 (2015-03-18), pages 25-26, XP055527256, Retrieved from the Internet: URL:http://www.sigmaaldrich.com/content/da m/sigma- aldrich/articles/biology/cloning-and-expre ssion-flag-and-3xflag/flag-and-3x-flag- overview.pdf

## Description

### CROSS REFERENCE

### FIELD OF THE DISCLOSURE

The present disclosure relates to systems for detecting at least two analytes in a sample.

### SEQUENCE LISTING

The instant application contains a Sequence Listing which has been submitted electronically in ASCII format and is hereby incorporated by reference in its entirety. Said ASCII copy, created on September 27, 2016, is named 013018-0008-228_SL.txt and is 2,227 bytes in size.

### BACKGROUND

Detection of analytes in samples is important in many industries including, for example, research, immunology, water quality assessment, environmental science and engineering, medicine, etc. See, for example, U.S. Patent No. 9,086,407 and U.S. Patent Application No. 15/222,376.

Different methods for detecting analytes in samples may be used, including, for example, high pressure liquid chromatography (HPLC), mass spectrometry, and enzyme linked immunosorbent assays (ELISA). While HPLC and mass spectrometry may be used to detect analytes on the basis of charge and/or size, ELISA may be used to detect an analyte based on antigens on the analyte that are recognizable by capture and detection agents (e.g. antibodies, aptamers, etc.), making it an important assay, especially in the life sciences. ELISA may be used to detect the presence, absence or the amount of an analyte in a sample.

While conventional ELISA is a widely used method, there is a continuing need to develop improved assays with lower cost and reduced assay time. First, conventional ELISA can require expensive capture and detection agents, such as antibodies. Thus, techniques with reduced antibody requirements would decrease expense of assays. In addition, conventional ELISA takes at least 2 hours to complete and generally includes at least 2 separate incubation and washing steps. Accordingly, it would be desirable to provide a method for detecting an analyte in a sample that takes less time and inputs to perform compared with conventional ELISA, while maintaining or improving the sensitivity of detection.
Schneider: "PerkinElmer Launches Innovative Multiplexing System AlphaPlex(TM) Reagent Technology Provides Life Sciences Researchers with More Data in Less Time" (2 September 2014) discloses the AlphaPlex^{(TM)} system.
Perkin Elmer: "AlphaPlex Assay Development Guide" (14 December 2015) discloses a guide to the AlphaPlex^{(TM)} system.
US 2003/232394 discloses a high throughput screening method and assay system for determining the interaction between C-reactive protein and components binding to it.
EGLEN et al.: "The use of AlphaScreen technology in HTS: current status.", Current Chemical Genomics, vol. 1, no. 1, 2008, pages 2-10 discloses the AlphaScreen technology.
WO2010/095033 discloses certain multiplex assay methods and compositions.
WO2012/129611 discloses methods and kits for detecting multiple analytes.

Reference to any prior art in this specification is not, and should not be taken as, an acknowledgment or any form of suggestion that this prior art forms part of the common general knowledge in any country.

### SUMMARY

The invention provides a system for detecting at least two analytes, comprising:
i) a first donor agent capable of binding to a first analyte;
ii) a first binder conjugated to a donor bead, said first binder capable of binding to the first donor agent;
iii) a tagged second donor agent capable of binding to a second analyte;
iv) a second binder conjugated to the donor bead, said second binder capable of binding to the tagged second donor agent;
v) a tagged first acceptor agent capable of binding to the first analyte, the tagged first acceptor agent comprising a plurality of covalently bound peptide tags;
vi) a first acceptor bead conjugated to an anti-peptide agent, the anti-peptide agent capable of binding with at least one of the plurality of covalently bound peptide tags;
vii) a second acceptor agent capable of binding with the second analyte; and
viii) a second acceptor bead capable of binding with the second acceptor agent,

wherein the first binder and the second binder are not the same type of binders, wherein the donor bead comprises a photosensitizer capable in its excited state of generating singlet oxygen and wherein each acceptor bead comprises a photoactive indicator capable of fluorescing upon reaction with singlet oxygen
and wherein the first and second acceptor beads emit a fluorescence at different wavelength.

In some embodiments, the anti-peptide agent comprises an affimer.

In some embodiments, the first donor agent comprises a first antibody.

In some embodiments, the second acceptor agent is a tagged second acceptor agent comprising a plurality of covalently bound second peptide tags.

In some embodiments, the donor bead is coated with streptavidin or a derivative thereof.

In some embodiments, the tagged first acceptor agent is a second antibody covalently bound to at least one peptide tag or a plurality of peptide tags to form a tagged second antibody.

In some embodiments, the tagged second antibody comprises an epitope of an anti-peptide antibody attached, conjugated or covalently bound to first acceptor bead.

In some embodiments, the donor bead is doped with a photosensitizer capable of emitting singlet oxygen when stimulated by light at a wavelength in the range of between 250-1100 nm, between 300-1000 nm, between 650-700 nm or between 660-680 nm.

In some embodiments, the first acceptor bead comprises a photoactive indicator that reacts with singlet oxygen forming a photoactive indicator capable of fluorescing, wherein irradiation of the photoactive indicator emits fluorescence at a wavelength in the range of between 500-625 nm, between 525-575 nm, between 525-550 nm or 540-560 nm.

In some embodiments, the donor bead and the first acceptor bead each have a diameter in the range of between 50-500 nm, between 100-500 nm, between 150-350 nm, between 250-350 nm or between 250-275 nm.

In certain embodiments, the second acceptor agent may be directly conjugated to the second acceptor bead. In certain embodiments, the second acceptor agent may be a tagged second acceptor agent comprising a plurality of covalently bound second peptide tags.

The system may, for example, be used in a method for detecting at least two different analytes in a sample, comprising: binding a first donor agent to a first analyte of the at least two analytes and separately binding a tagged first acceptor agent to the first analyte, wherein the tagged first acceptor agent comprises a plurality of covalently bound peptide tags. The method may further comprise: binding a tagged second donor agent to a second analyte of the at least two analytes and separately binding a second acceptor agent to the second analyte, attaching the first donor agent to a first binder conjugated on a donor bead and attaching the second donor agent to a second binder conjugated on the donor bead, binding at least one of the plurality of covalently bound peptide tags to an anti-peptide agent conjugated to a first acceptor bead, binding the second acceptor agent to a second acceptor bead, and detecting the at least two different analytes by sensing the product of an interaction between the donor bead and the first and second acceptor beads. The method may comprise: binding a tagged second donor agent to a second analyte of the at least two analytes and separately binding a second acceptor agent to the second analyte, wherein the second acceptor agent is directly conjugated to a second acceptor bead, attaching the first donor agent to a first binder conjugated on a donor bead and attaching the second donor agent to a second binder conjugated on the donor bead, binding at least one of the plurality of covalently bound peptide tags to an anti-peptide agent conjugated to a first acceptor bead, and detecting the at least two different analytes by sensing the product of an interaction between the donor bead and the first and second acceptor beads.

The system may, for example, be used in a method for detecting at least two epitopes of an analyte, comprising: binding a tagged donor agent to the analyte, attaching the tagged donor agent to a binder conjugated on a donor bead, and binding a tagged first acceptor agent to a first epitope of the analyte and separately binding a second acceptor agent to a second epitope of the analyte, wherein tagged first acceptor agent comprises a plurality of covalently bound peptide first tags. The second acceptor agent may be bound to a second acceptor bead. The method may further comprise: binding at least one of the plurality of covalently bound peptide tags to an anti-peptide first agent conjugated to a first acceptor bead. The method may further comprise: binding at least one of the plurality of covalently bound peptide tags to an anti-peptide first agent conjugated to a first acceptor bead, binding the second acceptor agent to a second acceptor bead, and detecting the at least two epitopes of the analyte by sensing the product of an interaction between the donor bead and the two acceptor beads.

In another embodiment, the analyte detection system may comprise 2 or more (for example 3 or more, or 4 or more) different types of acceptor beads each employing a photoactive indicator precursor that fluoresces at a different (or detectably different) wavelength. Each of the different types of acceptor beads may provide a different photoactive indicator. The different type of acceptor beads may comprise for example rubrene, europium (for example a europium chelate), samarium, or terbium, capable of reacting with singlet oxygen to produce a photoactive indicator capable of fluorescing.

In certain of the above embodiments, the anti-peptide second agent may bind to the donor (or photosensitive) bead while the tagged first agent is bound to the acceptor (or the photoactive indicator precursor) bead.

### DETAILED DESCRIPTION OF THE FIGURES

Figure 1: A graph of the Alpha Signal vs. pERK Lysate Concentration for Example 1 and Comparative Examples A&B.
Figure 2: A graph of the Signal to Background ratio vs. p-AKT S473 Lysate concentration for Examples 2-3 and Comparative Examples C&D.
Figure 3: A graph of the Alpha Signal vs. Insulin Concentration for Examples 4-11.
Figure 4. A graph of Alpha Signal vs. Wortmannin Concentration for Example 12.
Figure 5. A graph of Alpha Signal vs. Wortmannin Concentration for Example 13.
Figure 6. A graph of Alpha Signal vs. Insulin Concentration for Example 14.
Figure 7: Single analyte, 2 Epitope target detection system.
Figure 8: Dual analyte target detection system.

### DESCRIPTION OF THE EXEMPLARY EMBODIMENTS

Unless otherwise specified, any reference to a "donor" agent may refer to the first donor agent and/or the second donor agent, but the second donor agent must be a tagged agent. In certain embodiments, the donor agent may comprise, for example, an agent that may be capable of binding with the analyte, for example an antibody, inclusive, for example, a monoclonal antibody, a polyclonal antibody, a multivalent antibody, a chimeric antibody, a multispecific antibody, or an antibody fragment thereof; an aptamer; an affimer; a protein; a protein receptor; a protein receptor; a protein ligand; a fusion protein, comprising, for example, an immunoglobulin fusion partner, a fusion partner that stabilizes a receptor or a ligand, or a fusion partner that provides a target for binding; avidin, streptavidin, an avidin derivative, or a streptavidin derivative.

In certain embodiments, the donor agent may comprise at least one tag or a plurality of tags. The at least one tag or a plurality of tags that may be attached, conjugated, or covalently bound, to a donor agent may be a ligand. In certain embodiments, a plurality of tags comprising in the range of between 1-50 tags may be attached, such as conjugated or covalently bound, to a donor agent, for example, a plurality of tags comprising in the range of between 2-50 tags, such as 5-8, 3-7, 2-5, or such as 10-15, 15-20, or 25-50 tags, may be attached, conjugated, or covalently bound, to a donor agent. In certain embodiments, no more than 50 tags may be attached, such as conjugated or covalently bound, to a donor agent, for example, no more than 12 tags, such as no more than 10, no more than 8, no more than 7, no more than 6, no more than 5, or no more than 3 tags, may be attached, conjugated, or covalently bound, to a donor agent. In certain embodiments, one tag may be attached, such as conjugated or covalently bound, to a donor agent, for example, 2 tags, such as 3, 4, 5, 8, 10, or 12 tags, may be attached, conjugated, or covalently bound, to a donor agent. In certain embodiments, at least one of the plurality of tags of the tagged donor agent is a ligand, or the plurality of tags of the tagged donor agent are ligand tags, wherein the ligand tag is biotin, a biotin derivative, or a peptide tag.

Suitable ligands that may be attached, conjugated, or covalently bound, to a donor agent may include, but are not limited to biotin or derivatives thereof, such as 1-[4-azidosalicylamido]-6-[biotinamido]-hexane; psoralen-PEG3-biotin; or TFPA-PEG3-biotin; a metal chelate, such as copper, nickel, or cobalt; maleic anhydride; maleimide; or a peptide tag, such as a FLAG-tag. In certain embodiments, at least one of the plurality of tags of the tagged donor agent is a ligand, or the plurality of tags of the tagged donor agent, is or comprises an epitope of the binder attached, conjugated, or covalently bound, to a donor bead.

In certain embodiments, the ligand attached, conjugated to, or covalently bound to, a donor agent may comprise a peptide tag, a polyhistidine tag (His-tag), a HA-tag, a myc-tag, biotin or a derivative thereof including, for example, imino biotin, D-desthiobiotin, DSB-X-biotin, biotin dimers or arylstannylbiotin trimer. Biotin and derivatives thereof may be bound to the donor agent by biotinylation. Biotinylation reagents and methods for biotinylation of a target molecule are known in the art and include those described by Hermanson (Bioconjugate Techniques, Academic Press, 2008). Biotinylation may comprise, for example, primary amine biotinylation, sulfhydryl biotinylation, carboxyl biotinylation, or glycoprotein biotinylation. Methods for labeling proteins, RNA, DNA and other molecules with the above ligands are generally known in the art and may include methods described by Wong (Chemistry of Protein Conjugation and Cross-Linking, CRC Press LLC, 1991). In certain embodiments, the ligand attached, conjugated to, or covalently bound to, a donor agent, is or comprises a peptide tag.

For example, in certain embodiments, the peptide tag is a FLAG-tag is DYKDDDDK (SEQ ID NO: 1), a peptide tag having the amino acid sequence CDYKDDDDK (SEQ ID NO: 8), a FLAG octapeptide, or is a polypeptide protein tag, that can be attached, conjugated, or covalently bound, to the first antibody, or added to the first antibody, such as attached, conjugated, or covalently bound to the first antibody using Recombinant DNA technology. Suitable peptide tags that may be attached, conjugated, or covalently bound, to a first antibody may include, but are not limited to amino acid sequences having no more than 100 amino acids, for example, no more than 50 amino acids, no more than 30 amino acids, no more than 25 amino acids, such as no more than 20, no more than 15, no more than 12, no more than 10, no more than 9, or no more than 8 amino acids. In certain embodiments, peptide tags that may be attached, conjugated, or covalently bound, to a first antibody may have amino acid sequences in the range of between 5-15 amino acids, for example, in the range of between 6-12 amino acids, such as between 7-10, or between 8-9 amino acids. In certain embodiments, the peptide tag has the amino acid sequence DYKDDDDK (SEQ ID NO: 1) or CDYKDDDDK (SEQ ID NO: 8). In certain embodiments, peptide tags may not be naturally occurring. In certain embodiments, peptide tags may not be naturally occurring in an organism from which a sample is taken.

Unless otherwise specified, any reference to a "acceptor" agent may refer to the first acceptor agent and/or the second acceptor agent, but the first acceptor agent must be a tagged agent. The acceptor agent comprises an agent that may be capable of binding with the analyte, for example an antibody, inclusive, for example, a monoclonal antibody, a polyclonal antibody, a multivalent antibody, a chimeric antibody, a multispecific antibody, or an antibody fragment thereof; an aptamer; an affimer; a protein; a protein receptor; a protein ligand; or a fusion protein, comprising, for example, an immunoglobulin fusion partner, a fusion partner that stabilizes a receptor or a ligand, or a fusion partner that provides a target for binding.

In certain embodiments, the acceptor agent may comprise at least one peptide tag or a plurality of peptide tags covalently bound to the acceptor agent. In certain embodiments, the plurality of peptide tags covalently bound to the acceptor agent may be in the range of between 1-15 peptide tags, such as 2-9, 5-8, 3-7, 2-5, or 10-15, peptide tags covalently bound to the acceptor agent. In certain embodiments, no more than 15 peptide tags may be covalently bound to the acceptor agent, for example, no more than 12 peptide tags, such as no more than 10, no more than 8, no more than 7, no more than 6, no more than 5, or no more than 3 peptide tags, may be covalently bound to the acceptor agent.

Suitable peptide tags that may be covalently bound to a acceptor agent may include, but are not limited to amino acid sequences having no more than 30 amino acids, for example, no more than 25 amino acids, such as no more than 20, no more than 15, no more than 12, no more than 10, no more than 9, or no more than 8 amino acids. In certain embodiments, at least one, a plurality, or each, of the plurality of peptide tags that may be covalently bound to a second antibody may have amino acid sequences in the range of between 5-15 amino acids, for example, in the range of between 6-12 amino acids, such as between 7-10, or between 8-9 amino acids. In certain embodiments, at least one, a plurality, or each, of the plurality of peptide tags covalently bound to the second antibody is or comprises a FLAG-tag having the amino acid sequence DYKDDDDK (SEQ ID NO: 1), a peptide tag having the amino acid sequence CDYKDDDDK (SEQ ID NO: 8), a FLAG octapeptide, or is a polypeptide protein tag. In certain embodiments, for example, the peptide tag has the amino acid sequence DYKDDDDK (SEQ ID NO: 1), or the peptide tag has the amino acid sequence CDYKDDDDK (SEQ ID NO: 8). In certain embodiments, the protein tag, may be covalently bound to the second antibody by using Recombinant DNA technology. In certain embodiments, at least one of the plurality of peptide tags of the tagged second antibody, or the plurality of tags of the tagged second antibody, is or comprises an epitope of the anti-peptide antibody attached, conjugated, or covalently bound, to an acceptor bead, such as a second bead.

Suitable peptide tags that may be covalently bound to an acceptor agent may include, but are not limited to a FLAG-tag, for example DYKDDDDK (SEQ ID NO: 1); a peptide tag having the amino acid sequence KRITVEEALAHPYLEQYYDPTDE (SEQ ID NO: 2); a peptide tag having the amino acid sequence HHHHHH (SEQ ID NO: 3); a peptide tag having the amino acid sequence EQKLISEEDL (SEQ ID NO: 4); a peptide tag having the amino acid sequence YPYDVPDYA (SEQ ID NO: 5); a peptide tag having the amino acid sequence YTDIEMNRLGK (SEQ ID NO: 6); a peptide tag having the amino acid sequence QPELAPEDPED (SEQ ID NO: 7); a peptide tag having the amino acid sequence CDYKDDDDK (SEQ ID NO: 8) a FLAG octapeptide; a polypeptide protein tag; or a polypeptide sequence that does not include a plurality of consecutive amino acids with the same charge. In certain embodiments, at least a portion of the peptide tags may not be naturally occurring. In certain embodiments, the peptide tags do not denature or inactivate the peptide-tagged agent to which they are attached. In certain embodiments, at least one of the peptide tags is more hydrophilic than the FLAG-tag. In certain embodiments, at least a portion of the peptide tags may be removed from the peptide-tagged agent to which they are attached by treatment with the specific proteinase, enterokinase (enteropeptidase). In certain embodiments, at least a portion of the peptide tags may not be naturally occurring in an organism from which a sample is taken. In certain embodiments, peptide tags may include amino acid sequences having no more than 100 amino acids, for example, no more than 50, no more than 30, no more than 20, no more than 15, no more than 12, no more than 10, no more than 9, or no more than 8 amino acids. In certain embodiments, peptide tags may include amino acid sequences having in the range of 4-100 amino acids, for example in the range of 4-50, 4-30, 4-20, 6-15, 6-12, 8-20, 8-15, or in the range of 8-12 amino acids. In certain embodiments, the peptide tags may include one or more of the above features for example, the peptide tags may comprise no more than 15 amino acids, may not be naturally occurring, and/or may be more hydrophilic than the FLAG tag.

In certain embodiments, the peptide tags may also be used in conjunction with other affinity tags, for example a polyhistidine tag (His-tag), HA-tag or myc-tag. In certain further embodiments, for example, the ligand attached, conjugated to, or covalently bound to, a first agent, may comprise one or more of the above tags, for example one or more of the peptide tags may also be used in conjunction with one or more of polyhistidine tag (His-tag), HA-tag or myc-tag.

An agent used in an assay to bind with an analyte may be tagged with a peptide. For example, adding a peptide tag, such as a FLAG-tag, to the donor agent allows the donor agent to be bound and/or associated with an anti-peptide agent against the peptide tag (for example a peptide tag such as the FLAG-tag sequence). In certain embodiments, a peptide tag, such as a FLAG-tag, may also be used in conjunction with other affinity tags for example a polyhistidine tag (His-tag), HA-tag or myc-tag. The FLAG-tag was the first example of a fully functional epitope tag to be published in the scientific literature (see Hopp et al., Bio/Technology 6: 1204-1210, 1988). Its structure has been optimized for compatibility with the proteins it is attached to, in that it is more hydrophilic than other common epitope tags and therefore less likely to denature or inactivate proteins to which it is appended. In addition, it can be removed readily from proteins by treatment with the specific proteinase, enterokinase (enteropeptidase).

In addition to comprising a ligand attached, conjugated, or covalently bound, to an agent, such as a donor agent or an acceptor agent, at one or a plurality of sites, the agent must be capable of binding, capturing, or attaching, to the analyte, such as binding to an epitope of the analyte, for example a phospho-epitope of the analyte. For example, in certain embodiments, the tagged donor agent is capable of binding to a first epitope of the analyte and the acceptor agent is capable of binding to a second epitope of the analyte. In certain embodiments, the type of epitope of the first epitope of the analyte and the second epitope of the analyte are the same type of epitope, or are two different types of epitope. In certain embodiments, the donor agent and the acceptor agentare the same type of agent. In certain embodiments, the donor agent and the acceptor agent are different types of antibodies. In certain embodiments, the first epitope of the analyte and/or the second epitope of the analyte is a phospho-epitope. In certain embodiments, the first epitope of the analyte and the second epitope of the analyte do not overlap but are still in close proximity to each other, or do not overlap and are distal to each other. In certain embodiments, the first epitope of the analyte and the second epitope of the analyte do not overlap and are sufficiently distal to each such that no steric interactions or substantially no steric interactions, are incurred between the first and second agent. In certain embodiments, the epitopes are bound by the donor agent and the acceptor agent, respectively. In certain embodiments, the agent may comprise a naturally occurring antibody, or a mutant antibody, or may be protein containing the binding fragment of an antibody or a protein receptor.

The analyte detection system employs at least three types of beads, at least one donor bead and at least a first acceptor bead and a second acceptor bead. In certain embodiments, the ratio of the number of the at least one donor bead present is in the range of 0.1-10, for example in the range of between 0.1-0.2, between 0.2-0.5, between 0.5-1, between 1-2, between 2-5, or between 5-10, relative to the number of the first acceptor bead.

In certain embodiments, the ratio of the number of the at least one donor bead present is in the range of between 0.1-10, for example in the range of between 0.2-5, between 0.5-2, between 0.66-1.5, between 0.75-1.33, between 0.8-1.25, between 0.9-1.1, or between 0.95-1.05, relative to the number of the first acceptor bead. In certain embodiments, the ratio of the number of the at least one donor bead present is in the range of between 1-3, for example in the range of between 1.25-2.75, between 1.5-2.5, between 1.75-2.25, or between 1.95-2.05, relative to the number of the first acceptor bead.

In certain embodiments, the ratio of the number of the at least one donor bead present is in the range of 0.1-10, for example in the range of between 0.1-0.2, between 0.2-0.5, between 0.5-1, between 1-2, between 2-5, or between 5-10, relative to the number of the second acceptor bead. In certain embodiments, the ratio of the number of the at least one donor bead present is in the range of between 0.1-10, for example in the range of between 0.2-5, between 0.5-2, between 0.66-1.5, between 0.75-1.33, between 0.8-1.25, between 0.9-1.1, or between 0.95-1.05, relative to the number of the second acceptor bead. In certain embodiments, the ratio of the number of the at least one donor bead present is in the range of between 1-3, for example in the range of between 1.25-2.75, between 1.5-2.5, between 1.75-2.25, or between 1.95-2.05, relative to the number of the second acceptor bead. In certain embodiments, the ratio of the number of the at least one donor bead present is in the range of 0.1-10, for example in the range of between 0.1-0.2, between 0.2-0.5, between 0.5-1, between 1-2 (for example 2), between 2-5, or between 5-10, relative to the total of the at least a first acceptor bead and a second acceptor bead (for example the total of the number of a first, a second, a third, and a fourth acceptor bead). In certain embodiments, the ratio of the number of the at least one donor bead present is in the range of between 0.1-10, for example in the range of between 0.2-5, between 0.5-2, between 0.66-1.5, between 0.75-1.33, between 0.8-1.25, between 0.9-1.1, or between 0.95-1.05, relative to the total of the at least a first acceptor bead and a second acceptor bead. In certain embodiments, the ratio of the number of the at least one donor bead present is in the range of between 1-3, for example in the range of between 1.25-2.75, between 1.5-2.5, between 1.75-2.25, or between 1.95-2.05, relative to the total of the at least a first acceptor bead and a second acceptor bead.

The analyte detection system uses a 'donor' bead doped with a photosensitizer capable of emitting singlet oxygen; and first and second 'acceptor' beads doped with a photoactive indicator precursor capable of reacting with singlet oxygen to produce a photoactive indicator capable of fluorescing, wherein the first and second acceptor beads emit a fluorescence at different wavelength.
A 'donor' bead is capable of interacting with an 'acceptor' bead via a chemical transfer interaction, via emitting singlet oxygen. See, for example, U.S. Patent No. 5,807,675,. In certain embodiments, the beads may have a diameter in the range of between 20 nm and 20 mm, such as between 50-500 nm, between 100-500 nm, between 150-350 nm, 250-350 nm, 250-275 nm, between 280-310 nm, between 290-325 nm, between 310-350 nm, or between 325-350 nm. A first type of bead, a donor bead, may be capable of interacting with a second type of bead, an acceptor bead, via a chemical transfer interaction when the first type of bead and the second type of bead are in close proximity to each other, such as when the beads are disposed within the range of between 15-250 nm, for example, between 15-225 nm, between 25-225 nm, between 50-225 nm, between 100-225 nm, between 150-225 nm, between 100-200 nm, between 25-75 nm, between 50-100 nm, between 175-225 nm, between 190-210 nm, between 225-250 nm, or between 150-200 nm of one another, such as within 25 nm of one another, for example within 50 nm, within 75 nm, within 100 nm, within 125 nm, within 150 nm, within 175 nm, within 180 nm, within 190 nm, within 200 nm, within 210 nm, within 220 nm, within 230 nm, or within 240 nm of one another.

The analyte detection system may utilize two or more types of donor beads, such that each type of donor bead utilized may emit singlet oxygen when stimulated by light at the same wavelength. The analyte detection system may utilize two or more types of donor beads, such that light at the different wavelengths stimulates the different types of donor beads to emit singlet oxygen when stimulated, for example, light at a first wavelength stimulates a first type of donor bead to emit singlet oxygen, and light at a second wavelength stimulates a second type of donor bead to emit singlet oxygen. For example, in certain embodiments, the donor bead may emit singlet oxygen when stimulated by light, such as when stimulated by light at a wavelength in the range of between 250-1100 nm, for example, by light at a wavelength in the range of between 300-1000 nm, between 620-700 nm, between 620-650 nm, between 640-700 nm, between 650-700 nm, between 670-690 nm, between 680-700 nm, or between 660-680 nm, such as by light at a wavelength of 620 nm, 630 nm, 640 nm, 650 nm, 660 nm, 670 nm, 675 nm, 680 nm, 685 nm, 690 nm, or 700 nm. For example, the analyte detection system may utilize two or more types of donor beads, such that each type of donor bead utilized may emit singlet oxygen when stimulated by light at the same wavelength, such as at a wavelength in the range of between 650-700 nm, for example at 680 nm.

Acceptor beads that may be utilized in the analyte detection system, are a bead doped with a photoactive indicator precursor, such as rubrene, europium, a europium chelate, samarium, or terbium, capable of reacting with singlet oxygen to produce a photoactive indicator capable of fluorescing. The acceptor bead emits light, such as fluoresces, in response to a chemical transfer interaction with a donor bead, by an emission of singlet oxygen by the donor bead, when in close proximity to said donor bead. For example, the acceptor bead, such as a bead that is associated with the binding of least one of the plurality of peptide tags covalently bound to the acceptor agent be utilized in the analyte detection system comprises a photoactive indicator precursor that reacts with singlet oxygen forming a photoactive indicator. The analyte detection system utilizes two or more types of acceptor beads, such that when irradiated, the different types of acceptor beads emit a fluorescence at different wavelength, for example, when irradiated a first type of acceptor bead emits a fluorescence at a first wavelength, and when irradiated a second type of acceptor bead emits a fluorescence at a second wavelength. In certain embodiments, the acceptor bead may be irradiated by light at a wavelength in the range of between 250-1100 nm, for example, by light at a wavelength in the range of between 300-1000 nm, between 450-950 nm, between 360-441 nm, between 620-700 nm, between 600-630 nm, between 620-650 nm, between 640-700 nm, between 650-700 nm, between 670-690 nm, between 680-700 nm, or between 660-680 nm, such as by light at a wavelength of 620 nm, 630 nm, 640 nm, 650 nm, 660 nm, 670 nm, 675 nm, 680 nm, 685 nm, 690 nm, or 700 nm.

In certain embodiments, the photoactive indicator on the acceptor bead, when irradiated, may fluoresce (i.e., emit a fluorescence) at a wavelength in the range of between 500-625 nm, such as at a wavelength in the range of between 525-575 nm, between 525-550 nm, between 540-560 nm, between 540-550 nm, 590-620 nm, between 600-625 nm, or 610-620 nm, such as at a wavelength of 520 nm, 530 nm, 535 nm, 540 nm, 545 nm, 550 nm, 555 nm, 560 nm, 600 nm, 605 nm, 610 nm, 615 nm, 620 nm, or 625 nm. For example, when irradiated a first type of acceptor bead may emit a fluorescence at a first wavelength in the range of between 525-575 nm, such as at a first wavelength of 545 nm, and when irradiated a second type of acceptor bead may emit a fluorescence at a second wavelength in the range of between 590-625 nm, such as at a second wavelength of 615 nm.

Suitable binders, coated, attached, conjugated, or bound to a bead, for example a donor bead, may include, but are not limited to an antibody, inclusive, for example, a monoclonal antibody, a polyclonal antibody, a multivalent antibody, a chimeric antibody, a multispecific antibody, or an antibody fragment thereof; an aptamer; an affimer; a protein; a protein receptor; a protein receptor; a protein ligand; or a fusion protein, comprising, for example, an immunoglobulin fusion partner, a fusion partner that stabilizes a receptor or a ligand, or a fusion partner that provides a target for binding; avidin, streptavidin, an avidin derivative, or a streptavidin derivative. Derivatives of avidin or streptavidin are known in the art and may include forms of avidin or streptavidin that have been modified to increase their binding affinity to modified and/or unmodified solid substrates or ligands. For example, streptavidin may be modified to add one or more amine groups, histidine residues or sulfhydryl groups to the molecule. In certain embodiments, the derivative of streptavidin may comprise neutravidin, captavidin or streptavidin mutants (e.g. H127C or S139C). In certain embodiments, a hydrophobic or hydrophilic binder may be passively bound to the hydrophobic or hydrophilic solid substrates, respectively. For example, streptavidin (or derivates thereof) may be passively bound to a hydrophobic solid substrate. In certain embodiments, the solid substrate may comprise a linker which facilitates covalent bonding of the binder to the solid substrate. For example, the linker may comprise glutathione, maleic anhydride, a metal chelate, or maleimide. The binder may then be bound to the solid substrate via the linker.

In certain embodiments, the binder is an antibody having an affinity for at least one ligand attached, conjugated, or covalently bound, to an antibody, such as a first antibody. For example, the further antibody binder may have a selective affinity for binding to the at least one, or each of the plurality of ligands attached, conjugated, or covalently bound, to an antibody, such as a first antibody. For example, in certain embodiments, the binder may be a further antibody, such as a second anti-peptide antibody having a selective affinity for at least one peptide tag, or a plurality of peptide tags attached, conjugated, or covalently bound, to a first antibody.

In certain embodiments, the binder is protein A and/or protein G that is coated on the first bead. For example, in certain embodiments, the donor bead may be coated with protein A and/or protein G, which are suitable for attaching to an antibody, in a solution. In certain embodiments, however, protein A and/or protein G may provide reduced utility in samples containing endogenous antibodies, such as serum or plasma, as these will block binding of the desired agent(s).

In certain embodiments, the anti-peptide agent coated, attached, conjugated, or bound to a bead, for example, a donor bead or an acceptor bead, may include, but are not limited to an antibody comprising a binding region highly selective for the amino acid sequence, or a portion thereof, of the peptide tag covalently bound to the second agent, such as a peptide tag having the amino acid sequence DYKDDDDK (SEQ ID NO: 1) or CDYKDDDDK (SEQ ID NO: 8). For example, the binding region of the anti-peptide agent may be highly selective for at least 60% of amino acid sequence of the peptide tag covalently bound to the second agent, such as 70%, 80%, 90%, 95%, or 100%, of amino acid sequence of the peptide tag covalently bound to the second agent.

In some embodiments, the donor bead or the acceptor bead may be treated with a blocking agent that binds non-specifically to and saturates binding sites to prevent direct binding of unwanted components (e.g. the analyte, non-analyte antibodies present in a sample) to the excess sites on the first or second bead. Examples of blocking agents may include, for example, gelatin, BSA, egg albumin, casein, and non-fat milk. In some embodiments, the solid substrate and/or the bound immobilisation agent may be treated with the blocking agent prior to the addition of the capture agent or concurrent with the addition of the capture agent.

Detecting the presence of the analyte is by sensing an emission resulting from an interaction between the attached donor bead and the bound acceptor bead.

The binding an analyte with a donor agent and an acceptor agent forms an analyte complex, and the resulting analyte complex is then immobilized to two different types of beads, comprising an acceptor bead, and a donor bead. For example, the system may be used by attaching at least one of the one or more first tags to a binder conjugated to a donorbead, and binding at least one of the plurality of covalently bound peptide second tags to an antibody conjugated to a acceptor bead. The resulting analyte complex, now attached to a donor bead and bound to an acceptor bead, is available for detecting the presence of the analyte by sensing an emission resulting from an interaction between the attached donor bead and the bound acceptor bead. For example, the donor bead, now in close proximity to the acceptor bead via the attached and bound analyte complex, and having a photosensitizer contained within the bead may emit singlet oxygen when irradiated with light. The singlet oxygen produced from the donor bead may react with a photoactive indicator precursor contained within the acceptor bead because of its close proximity to produce a photoactive indicator that is capable of fluorescing when irradiated with light.

The detection method may further include exciting a photosensitizer contained within or coated on a donor bead by irradiation with light to produce singlet oxygen that reacts with a photoactive indicator precursor contained within or coated on an acceptor bead to produce said photoactive indicator within or on the acceptor bead. The detection method may further include irradiating the produced photoactive indicator within or on the acceptor bead and measuring the fluorescence emitted by said photoactive indicator.

The system allows detecting at least two different analytes in a sample, which may include detecting at least two epitopes of an analyte in a sample. For example, in certain embodiments, the absence of a specific analyte is determined when no fluorescence within a detection range is sensed upon irradiation of the donor bead and the acceptor bead.

Methods of using the system may include mixing the sample, the tagged first agent, the tagged second agent, and at least 2 or a plurality of acceptor beads, followed by adding at least one or a plurality of donor beads. The methods may include incubating the sample, the tagged first agent, the tagged second agent, and at least 2 or a plurality of acceptor beads for approximately 1 hour, followed by adding at least one or a plurality of donor beads, and further incubating for approximately 1 hour. The addition of the at least 2 or a plurality of acceptor beads and the at least one or a plurality of donor beads to the mixture may be simultaneously, or sequentially. The donor bead may be added to the mixture, prior to, concurrently with, or after the at least 2 acceptor beads, and the resulting mixture is incubated for a period of time, for example, for less than 30 minutes, for less than one hour, for approximately one hour, for more than one hour, for less than two hours. The method comprises mixing the donor bead with the incubated mixture, in the presence or absence of the at least 2 acceptor beads, and incubating the resulting mixture for a period of time, for example, for less than 30 minutes, for less than one hour, for approximately one hour, for more than one hour, for less than two hours. The donor bead may be added to the mixture, prior to, concurrently with, or after the at least 2 acceptor beads, and the resulting mixture is incubated for a period of time, for example, for less than 30 minutes, for less than one hour, for approximately one hour, for more than one hour, for less than two hours. The method may comprises mixing the donor bead with the incubated mixture, in the presence or absence of the at least 2 acceptor beads, and incubating the resulting mixture for a period of time, for example, for less than 30 minutes, for less than one hour, for approximately one hour, for more than one hour, for less than two hours. The sample, the tagged first agent, the tagged second agent, and the at least 2 acceptor beads, in the presence or the absence of the donor bead, may be incubated for a period of time, for example, for approximately one hour, for less than one hour, for greater than one hour, for less than two hours, or for approximately two hours.

When using the system, the concentration of the binder attached, conjugated, or covalently bound to the donor bead, may, for example, be at least as large as the concentration of the tagged first agent in the mixture. The concentration of the binder attached, conjugated, or covalently bound to the donor bead, may, for example, be at least 2 times greater than the concentration of the tagged first agent in the mixture, for example, the concentration of the binder is at least 3 times greater, such as at least 4 times greater, at least 5 times greater, at least 7 times greater, at least 9 times greater, or at least 10 times greater than the concentration of the tagged first agent in the mixture. The concentration of the binder attached, conjugated, or covalently bound to the donor bead, may, for example, be in the range of between 2-15 times greater than the concentration of the tagged first agent in the mixture, for example, the concentration of the binder is in the range of between 2-10 times greater, such as between 2-8 times greater, between 2-5 times greater, between 4-12 times greater, between 3-9 times greater, between 4-8 times greater, or between 10-15 times greater, than the concentration of the tagged first agent in the mixture.

The binder attached, conjugated, or covalently bound to the donor bead may, e.g. be present at a ratio of at least 100:1, 125:1, 150:1, 200:1, 250:1, 300:1, 400:1, 500:1, 750:1, or 1000:1, on a weight:weight basis, relative to the tagged first agent in the mixture. The binder may, e.g., be present at a ratio of in a range of 100:1-1000:1, 100:1-500:1, 100:1-300:1, 250:1-300:1, 300:1-1000:1, 300:1-500:1, 300:1-400:1, 300:1-350:1, 325:1-375:1, 300:1-310:1, 310:1-320:1, 320:1-330:1, 330:1-340:1, 340:1-350:1, or 350:1-360:1, on a weight:weight basis, relative to the tagged first agent in the mixture.

The binder attached, conjugated, or covalently bound to the donor bead may, e.g., be present at a ratio of at least 100:1, 125:1, 150:1, 200:1, 250:1, 300:1, 400:1, 500:1, 750:1, or 1000:1, on a mole:mole basis, relative to the tagged first agent in the mixture. The binder may, e.g., be present at a ratio of in a range of 100:1-1000:1, 100:1-500:1, 100:1-300:1, 250:1-300:1, 300:1-1000:1, 300:1-500:1, 300:1-400:1, 300:1-350:1, 325:1-375:1, 300:1-310:1, 310:1-320:1, 320:1-330:1, 330:1-340:1, 340:1-350:1, or 350:1-360:1, on a mole:mole basis, relative to the tagged first agent in the mixture.

The concentration of the anti-peptide agent, such as the anti-peptide agent attached, conjugated, or covalently bound to the acceptor bead, may, for example, be at least as large as the concentration of the tagged first agent in the mixture. The concentration of the anti-peptide agent, such as the anti-peptide agent attached, conjugated, or covalently bound to the acceptor bead, may, for example, be at least 2 times greater than the concentration of the tagged second agent in the mixture, for example, the concentration of the anti-peptide agent is at least 3 times greater, such as at least 4 times greater, at least 5 times greater, at least 7 times greater, at least 9 times greater, or at least 10 times greater than the concentration of the tagged second agent in the mixture. The concentration of the anti-peptide agent, such as the anti-peptide agent attached, conjugated, or covalently bound to the acceptor bead, may, for example, be in the range of between 2-15 times greater than the concentration of the tagged second agent in the mixture, for example, the concentration of the anti-peptide agent is in the range of between 2-10 times greater, such as between 2-8 times greater, between 2-5 times greater, between 4-12 times greater, between 3-9 times greater, between 4-8 times greater, or between 10-15 times greater, than the concentration of the tagged second agent in the mixture.

The anti-peptide agent may, e.g., be present at a ratio of at least 100:1, 125:1, 150:1, 200:1, 250:1, 300:1, 400:1, 500:1, 750:1, or 1000:1, on a weight:weight basis, relative to the tagged second agent in the mixture. The binder may, e.g., be present at a ratio of in a range of 100:1-1000:1, 100:1-500:1, 100:1-300:1, 250:1-300:1, 300:1-1000:1, 300:1-500:1, 300:1-400:1, 300:1-350:1, 325:1-375:1, 300:1-310:1, 310:1-320:1, 320:1-330:1, 330:1-340:1, 340:1-350:1, or 350:1-360:1, on a weight:weight basis, relative to the tagged second agent in the mixture.

The anti-peptide agent may, e.g., be present at a ratio of at least 100:1, 125:1, 150:1, 200:1, 250:1, 300:1, 400:1, 500:1, 750:1, or 1000:1, on a mole:mole basis, relative to the tagged second agent in the mixture. The binder may, e.g., be present at a ratio of in a range of 100:1-1000:1, 100:1-500:1, 100:1-300:1, 250:1-300:1, 300:1-1000:1, 300:1-500:1, 300:1-400:1, 300:1-350:1, 325:1-375:1, 300:1-310:1, 310:1-320:1, 320:1-330:1, 330:1-340:1, 340:1-350:1, or 350:1-360:1, on a mole:mole basis, relative to the tagged second agent in the mixture.

The concentration of the anti-peptide agent, such as the anti-peptide agent attached, conjugated, or covalently bound to a donor bead or a acceptor bead, is, e.g., less than 5000 nanograms of the anti-peptide agent per milliliter of solution, for example, less than 2500 ng/ml, less than 2000 ng/ml, less than 1500 ng/ml, less than 1000 ng/ml, less than 500 ng/ml, less than 400 ng/ml, less than 300 ng/ml, less than 250 ng/ml, less than 200 ng/ml, less than 100 ng/ml, or less than 50 ng/ml. The concentration of the anti-peptide agent, such as the anti-peptide agent attached, conjugated, or covalently bound to the acceptor bead, is, e.g. in the range of between 1-500 ng/ml, for example, between 1-400 ng/ml, between 1-300 ng/ml, between 1-250 ng/ml, between 1-200 ng/ml, between 1-100 ng/ml, or between 1-50 ng/ml. The concentration of the anti-peptide agent, such as the anti-peptide agent attached, conjugated, or covalently bound to a donor bead or a acceptor bead, is, e.g. a concentration of up to 10 µg/ml, for example, up to 1 µg/ml, 5 µg/ml, or 8 µg/ml.

The concentration of the analyte is, e.g., in the range of between 1-100 ng/ml, for example, between 1-50 ng/ml, between 1-25 ng/ml, between 10-50 ng/ml, between 1-10 ng/ml, e.g., below 1 ng/mL; or above 50 ng/mL; or 100 ng/ml or less, 10 ng/ml or less, 1 ng/ml or less, 100 pg/ml or less, or 10 pg/ml or less, 1 pg/ml or less, 100 fg/ml or less, 10 fg/ml or less, or 1 fg/ml or less; or greater than 10 ng/ml, greater than 1 ng/ml, greater than 100 pg/ml or, greater than 10 pg/ml, greater than 1 pg/ml, greater than 100 fg/ml, greater than 10 fg/ml or greater than 1 fg/ml; or is between 1 fg/ml to 100 ng/ml, 1 fg/ml to 10 ng/ml, 1 fg/ml to 1 ng/ml, 10 fg/ml to 100 ng/ml, 10 fg/ml to 10 ng/ml, 10 fg/ml to 1 ng/ml, 100 fg/ml to 100 ng/ml, 100 fg/ml to 10 ng/ml, 100 fg/ml to 1 ng/ml, 1 pg/ml to 100 ng/ml, 1 pg/ml to 10 ng/ml, or 1 pg/ml to 1 ng/ml.

In certain embodiments, the affinity, or rate constant, for binding of the binder to the first agent is approximately the same as the affinity, or rate constant, for binding of the binder to any of the non-analyte antibodies. In certain embodiments, the affinity, or rate constant, for binding of the binder to the first agent is at least 2 times larger than the affinity, or rate constant, for binding of the binder to any of the non-analyte antibodies. For example, the affinity, or rate constant, for binding of the binder to the first agent is at least 5 times larger, such as at least 10 times larger, at least 25 times larger, at least 50 times larger, at least 75 times larger, or at least 100 times larger than the affinity, or rate constant, for binding of the binder to any of the non-analyte antibodies.

In certain embodiments, the affinity, or rate constant, for binding of the anti-peptide agent to the tagged second agent is approximately the same as the affinity, or rate constant, for binding of the binder to any of the non-analyte antibodies. In certain embodiments, the affinity, or rate constant, for binding of the anti-peptide agent to the tagged second agent is at least 2 times larger than the affinity, or rate constant, for binding of the binder to any of the non-analyte antibodies. For example, the affinity, or rate constant, for binding of the anti-peptide agent to the tagged second agent is at least 5 times larger, such as at least 10 times larger, at least 25 times larger, at least 50 times larger, at least 75 times larger, or at least 100 times larger than the affinity, or rate constant, for binding of the binder to any of the non-analyte antibodies.

In certain embodiments, the dissociation constant for binding of the anti-peptide agent to the tagged second agent is approximately the same as the dissociation constant for binding of the binder to any of the non-analyte antibodies. In certain embodiments, the dissociation constant for binding of the anti-peptide agent to the tagged second agent is at least 2 times smaller than the dissociation constant for binding of the binder to any of the non-analyte antibodies. For example, the dissociation constant for binding of the anti-peptide agent to the tagged second agent is at least 5 times smaller, such as at least 10 times smaller, at least 25 times smaller, at least 50 times smaller, at least 75 times smaller, or at least 100 times smaller than the dissociation constant for binding of the binder to any of the non-analyte antibodies.

The plurality of tagged first agents and/or the plurality of second agents may, e.g., be stored at a predetermined temperature, for example, at 4 °C, room temperature, less than 10 °C, less than 20 °C, or less than 30 °C.

The tagged first agents may, e.g., be stored in or introduced into a first liquid buffer and the tagged second agents may, e.g., be stored in or introduced into a second liquid buffer.

The beads, such as the donor bead or the acceptor bead, may, e.g. be stored under low light conditions, such as in a sealed container. The donor beads may, e.g., be stored under low light conditions, such as in a sealed container.

### Examples

### EXAMPLE 1 - Comparison of Analyte Detection Using Peptide/Anti-Peptide and Direct Conjugation Systems.

A series of pERK analyte detection experiments were conducted employing a biotinylated anti-pERK IgG under comparable conditions. In Example 1 (the peptide/anti-peptide experiment), anti-total ERK IgG was tagged with a plurality of amino acid peptide tags, specifically CDYKDDDDK (SEQ ID NO: 8), and the acceptor beads were conjugated with mouse monoclonal IgG1 anti-peptide agent. In Comparative Examples A and B, the anti-total ERK IgG was directly conjugated onto the acceptor beads. The concentrations of the anti-total ERK IgG were as noted in Table 1 while all other variables were held constant.

**Table 1**

| **Example** | **Concentration of Anti-Total ERK IgG (ng/ml of sample)** |
|---|---|
| Example 1 | 250 |
| Comparative Example A | 2500 |
| Comparative Example B | 500 |

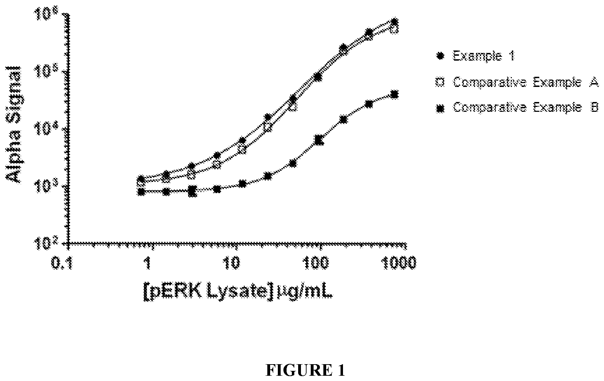

Following incubation, the biotinylated anti-pERK IgG was bound to streptavidin-coated photosensitizer donor beads while, in Example 1, the tagged anti-total ERK IgG was additionally bound via the mouse monoclonal IgG1 anti-peptide agent to the photoactive precursor acceptor beads. The amount of signal detected for each example is presented in FIG. 1.

### EXAMPLES 2-3 - Effect of Non-Analyte Antibodies.

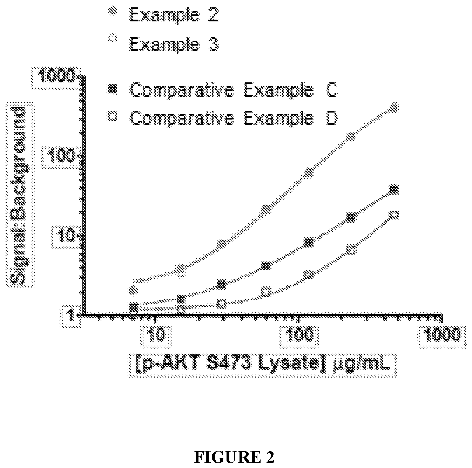

A series of experiments were conducted on samples of protein lysates of serum-activated HEK-293 cells in the absence of any non-analyte antibodies and again in the presence of non-analyte rabbit antibodies at a fixed concentration of 10 mg/ml and analyzed for p-AKT 1/2/3 (Ser473) analyte under comparable conditions and concentrations. Example 2 (no non-analyte antibody) and 3 (spiked with non-analyte antibody) were conducted employing a peptide tagged antibody with complementary antibody conjugated on the acceptor beads and Comparative Examples C (no non-analyte antibody) and D (spiked with non-analyte antibody) repeated the same assays but employed an untagged antibody and protein A-coated acceptor beads. The signal to background signal ratios recorded are presented in FIG. 2.

### EXAMPLES 4-11 - Detection of Multiple Analytes.

Phosphoprotein and total protein analytes were assayed in triplicate from a single lysate sample by delivering portions of the sample to individual culture wells of an assay plate and using biotinylated antibodies and peptide-tagged antibodies to bind the analyte. Table 2 lists the protein detected in each of Examples 4-11.

**Table 2**

| **Example** | **Target** |
|---|---|
| Example 4 | AKT (pS473) |
| Example 5 | AKT (pT308) |
| Example 6 | ERK (pT202 Y204) |
| Example 7 | P70S6K (pT389) |
| Example 8 | eIF4E (pS209) |
| Example 9 | CREB (pS133) |
| Example 10 | Total AKT 1 |
| Example 11 | Total ERK |

During incubation, the peptide-tagged antibodies, tagged with a plurality of tags each having the amino acid sequence CDYKDDDDK (SEQ ID NO: 8), were bound with mouse monoclonal IgG1 anti-peptide antibodies conjugated to photoactive precursor acceptor beads while the biotinylated antibodies were bound to streptavidin-coated photosensitizer donor beads.

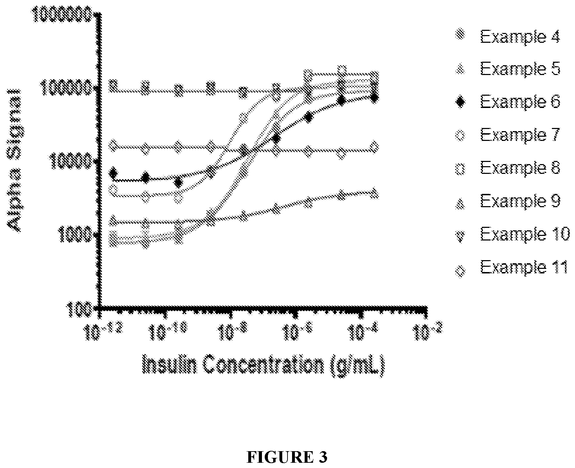

To produce the cells for the lysate sample, individual MCF-7 cells were plated overnight at 200K cells/ml in 200 ml MEM + 10% FCS. Cells were then serum starved using MEM + 0% FCS for 2 hours, and stimulated for 30 minutes with a dose range of insulin. The amount of signal detected in each of Examples 4-11 is presented in FIG. 3.

### EXAMPLE 12 - Multiplex Detection of Multiple Epitopes.

A cell lysate was prepared as follows: MCF-7 cells were plated overnight at 200K/ml in 200 mL MEM + 10% FCS. Cells were then treated for 2 hours with varying concentrations of wortmannin in MEM +1% FBS, and then stimulated for 30 min with a dose range of insulin, and then lysed.

The cell lysate was incubated in a multi-well plate with the components listed in Table 3 to measure AKT (pS473) and total AKT1 protein targets.

**Table 3**

| **Assay Components** |
|---|
| • Anti-AKT (pS473) antibodies bound to Europium acceptor beads |
| • Peptide-tagged anti-total AKT1 antibodies |
| • Mouse monoclonal IgG1 anti-peptide antibodies bound to Terbium acceptor beads |
| • Biotinylated anti-total AKT1 antibodies |
| • Streptavidin-coated photosensitizer donor beads |

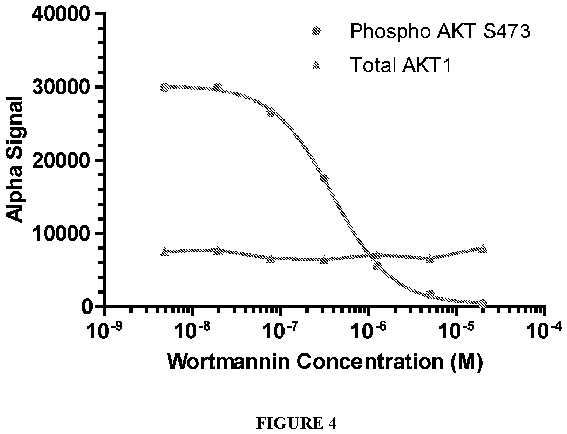

Detection signals for AKT (pS473) and total AKT1 in the incubated cell lysate were measured using a plate reader at the indicated wortmannin concentrations as shown in FIG. 4.

### EXAMPLE 13 - Multiplex Detection of Multiple Analytes.

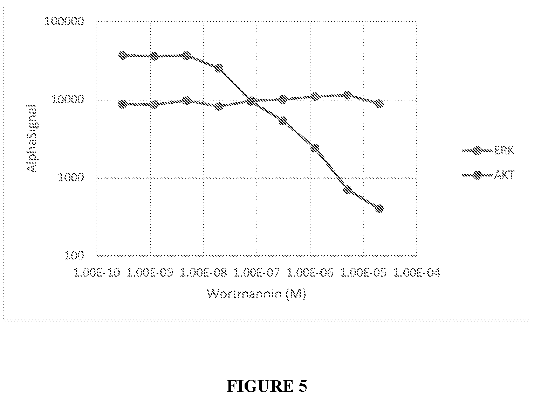

A cell lysate was prepared as follows: MCF7 cells were plated overnight at 40K cells/well in a 96 well plate in 10% fetal bovine serum at 37 °C in 95% air/5% CO2. Cells were then treated with wortmannin for 2 hours at the concentrations shown in FIG. 5, and stimulated for 10 minutes with a dose range of insulin, and then lysed.

The cell lysate was incubated in a multi-well plate with the components listed in Table 4 to measure lysate AKT (pS473) and ERK (pT202 Y204) protein targets.

**Table 4**

| **Assay Components** |
|---|
| • Anti-AKT (pS473) antibodies directly conjugated to Europium acceptor beads |
| • Peptide-tagged anti-ERK (pT202 Y204) antibodies |
| • Mouse monoclonal IgG1 anti-peptide antibodies bound to Terbium acceptor beads |
| • Biotinylated anti-total ERK antibodies |
| • Streptavidin-coated photosensitizer donor beads |
| • Biotinylated anti-total AKT1 antibodies |

Detection signals for AKT (pS473) and ERK (pT202 Y204) in the incubated cell lysate were measured using a plate reader as shown in FIG. 5.

### EXAMPLE 14 - Multiplex Detection of Multiple Analytes.

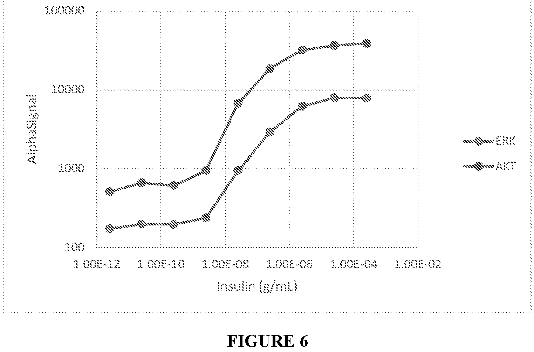

A cell lysate was prepared as follows: MCF7 cells were plated overnight at 40K cells/well in a 96 well plate in 10% fetal bovine serum at 37 °C in 95% air/5% CO2. Cells were then serum starved for 2 hours and treated with insulin for 10 minutes at the concentrations shown in FIG. 6, and then lysed.

The cell lysate was incubated in a multi-well plate with the components listed above in Table 4 to measure lysate AKT (pS473) and ERK (pT202 Y204) protein targets.

Detection signals for AKT (pS473) and ERK (pT202 Y204) in the incubated cell lysate were measured using a plate reader as shown in FIG. 6.

### Prophetic Examples:

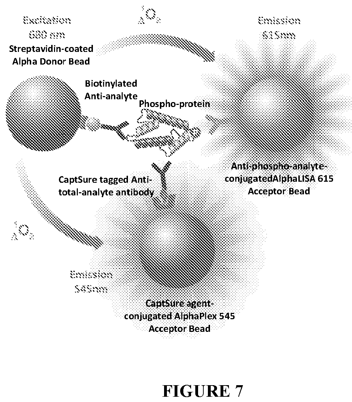

As illustrated in FIG. 7, a single target analyte detection system comprising an AlphaLISA 615 Acceptor bead™ directly-conjugated with an antibody to a phosphorylated site on the target protein, wherein the AlphaPlex 545 Acceptor bead™ is coated with the CaptSure agent™, which binds the CaptSure tagged anti-total antibody, and wherein the Alpha Donor bead binds the biotinylated anti-total antibody.

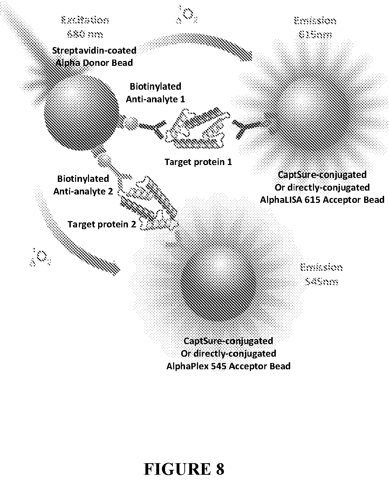

As illustrated in FIG. 8, a dual target analyte detection system comprising an AlphaLISA 615 Acceptor bead™ and the AlphaPlex 545 Acceptor bead™ each binding distinct proteins via specific antibodies associated with the bead, wherein one of the Acceptor beads will be directly conjugated to the antibody, and the other will bind the antibody via the CaptSure agent™, and wherein the type of linkage will depend on the specific AlphaPlex SureFire Ultra kit being used.

## Claims

1. A system for detecting at least two analytes, comprising:
i) a first donor agent capable of binding to a first analyte;
ii) a first binder conjugated to a donor bead, said first binder capable of binding to the first donor agent;
iii) a tagged second donor agent capable of binding to a second analyte;
iv) a second binder conjugated to the donor bead, said second binder capable of binding to the tagged second donor agent;
v) a tagged first acceptor agent capable of binding to the first analyte, the tagged first acceptor agent comprising a plurality of covalently bound peptide tags;
vi) a first acceptor bead conjugated to an anti-peptide agent, the anti-peptide agent capable of binding with at least one of the plurality of covalently bound peptide tags;
vii) a second acceptor agent capable of binding with the second analyte; and
viii) a second acceptor bead capable of binding with the second acceptor agent,
wherein the first binder and the second binder are not the same type of binders, wherein the donor bead comprises a photosensitizer capable of emitting singlet oxygen and wherein each acceptor bead comprises a photoactive indicator capable of fluorescing upon reaction with singlet oxygen
and wherein the first and second acceptor beads emit a fluorescence at different wavelength.

2. The system of claim 1, wherein the anti-peptide agent comprises an affimer.

3. The system of claim 1, wherein the first donor agent comprises a first antibody.

4. The system of claim 3, wherein the second acceptor agent is a tagged second acceptor agent comprising a plurality of covalently bound second peptide tags.

5. The system of any one of claims 1-4, wherein the donor bead is coated with streptavidin or a derivative thereof.

6. The system of claim 1, wherein the tagged first acceptor agent is a second antibody covalently bound to at least one peptide tag or a plurality of peptide tags to form a tagged second antibody.

7. The system of claim 6, wherein the tagged second antibody comprises an epitope of an anti-peptide antibody attached, conjugated or covalently bound to first acceptor bead.

8. The system of claim 1, wherein the donor bead is doped with a photosensitizer capable of emitting singlet oxygen when stimulated by light at a wavelength in the range of between 250-1100 nm, between 300-1000 nm, between 650-700 nm or between 660-680 nm.

9. The system of claim 1, wherein the first acceptor bead comprises a photoactive indicator that reacts with singlet oxygen forming a photoactive indicator capable of fluorescing, wherein irradiation of the photoactive indicator emits fluorescence at a wavelength in the range of between 500-625 nm, between 525-575 nm, between 525-550 nm or 540-560 nm.

10. The system of claim 1, wherein the donor bead and the first acceptor bead each have a diameter in the range of between 50-500 nm, between 100-500 nm, between 150-350 nm, between 250-350 nm or between 250-275 nm.

## Patentansprüche

1. System zum Detektieren von mindestens zwei Analyten, umfassend:
i) ein erstes Donormittel, das in der Lage ist,, an einen ersten Analyten zu binden;
ii) ein erstes Bindemittel, das an ein Donorkügelchen konjugiert ist, wobei das erste Bindemittel in der Lage ist, an das erste Donormittel zu binden;
iii) ein getaggtes zweites Donormittel, das in der Lage ist, an einen zweiten Analyten zu binden;
iv) ein zweites Bindemittel, das an das Donorkügelchen konjugiert ist, wobei das zweite Bindemittel in der Lage ist, an das getaggte zweite Donormittel zu binden;
v) ein getaggtes erstes Akzeptormittel, das in der Lage ist, an den ersten Analyten zu binden, wobei das getaggte erste Akzeptormittel eine Vielzahl von kovalent gebundenen Peptid-Tags umfasst;
vi) ein erstes Akzeptorkügelchen, das an ein Anti-Peptidmittel konjugiert ist, wobei das Anti-Peptidmittel in der Lage ist, mit mindestens einem der Vielzahl von kovalent gebundenen Peptid-Tags zu binden;
vii) ein zweites Akzeptormittel, das in der Lage ist, mit dem zweiten Analyten zu binden; und
viii) ein zweites Akzeptorkügelchen, das in der Lage ist, mit dem zweiten Akzeptormittel zu binden,
wobei das erste Bindemittel und das zweite Bindemittel nicht derselbe Typ von Bindemittel sind,
wobei das Donorkügelchen einen Photosensibilisator umfasst, der in der Lage ist, Singulett-Sauerstoff zu emittieren und wobei jedes Akzeptorkügelchen einen photoaktiven Indikator umfasst, der in der Lage ist, nach Reaktion mit Singulett-Sauerstoff zu fluoreszieren
und wobei das erste und zweite Akzeptorkügelchen Fluoreszenz bei verschiedener Wellenlänge emittieren.

2. System nach Anspruch 1, wobei das Anti-Peptidmittel einen Affimer umfasst.

3. System nach Anspruch 1, wobei das erste Donormittel einen ersten Antikörper umfasst.

4. System nach Anspruch 3, wobei das zweite Akzeptormittel ein getaggtes zweites Akzeptormittel ist, das eine Vielzahl von kovalent gebundenen zweiten Peptid-Tags umfasst.

5. System nach einem der Ansprüche 1-4, wobei das Donorkügelchen mit Streptavidin oder einem Derivat davon beschichtet ist.

6. System nach Anspruch 1, wobei das getaggte zweite Akzeptormittel ein zweiter Antikörper ist, der kovalent an mindestens ein Peptid-Tag oder eine Vielzahl von Peptid-Tags gebunden ist, um einen getaggten zweiten Antikörper zu bilden.

7. System nach Anspruch 6, wobei der getaggte zweite Antikörper ein Epitop eines Anti-Peptid-Antikörpers, angebracht, konjugiert oder kovalent gebunden an das erste Akzeptorkügelchen umfasst.

8. System nach Anspruch 1, wobei das Donorkügelchen mit einem Photosensibilisator dotiert ist, der in der Lage ist, Singulett-Sauerstoff zu emittieren, wenn er mit Licht bei einer Wellenlänge im Bereich zwischen 250-1100 nm, zwischen 300-1000 nm, zwischen 650-700 nm oder zwischen 660-680 nm stimuliert wird.

9. System nach Anspruch 1, wobei das erste Akzeptorkügelchen einen photoaktiven Indikator umfasst, der mit Singulett-Sauerstoff reagiert, einen photoaktiven Indikator bildet, der in der Lage ist, zu fluoreszieren, wobei eine Bestrahlung des photoaktiven Indikators Fluoreszenz bei einer Wellenlänge im Bereich zwischen 500-625 nm, zwischen 525-575 nm, zwischen 525-550 nm oder 540-560 nm emittiert.

10. System nach Anspruch 1, wobei das erste Donorkügelchen und das erste Akzeptorkügelchen jeweils einen Durchmesser im Bereich zwischen 50-500 nm, zwischen 100-500 nm, zwischen 150-350 nm, zwischen 250-350 nm oder zwischen 250-275 nm aufweist.

## Revendications

1. Système de détection d'au moins deux analytes, comprenant :
i) un premier agent donneur capable de se lier à un premier analyte ;
ii) un premier liant conjugué à une bille donneuse, ledit premier liant étant capable de se lier au premier agent donneur ;
iii) un deuxième agent donneur marqué capable de se lier à un deuxième analyte ;
iv) un deuxième liant conjugué à la bille donneuse, ledit deuxième liant étant capable de se lier au deuxième agent donneur marqué ;
v) un premier agent accepteur marqué capable de se lier au premier analyte, le premier agent accepteur marqué comprenant une pluralité de marqueurs peptidiques liés de manière covalente ;
vi) une première bille acceptrice conjuguée à un agent anti-peptide, l'agent anti-peptide étant capable de se lier à au moins l'un de la pluralité de marqueurs peptidiques liés de manière covalente ;
vii) un deuxième agent accepteur capable de se lier au deuxième analyte ; et
viii) une deuxième bille acceptrice capable de se lier au deuxième agent accepteur,
dans lequel le premier liant et le deuxième liant ne sont pas du même type de liants,
dans lequel la bille donneuse comprend un photosensibilisateur capable d'émettre de l'oxygène singulet et où chaque bille acceptrice comprend un indicateur photoactif capable de devenir fluorescent lors de la réaction avec l'oxygène singulet
et dans lequel les première et deuxième billes acceptrices émettent une fluorescence à différentes longueurs d'onde.

2. Système selon la revendication 1, dans lequel l'agent anti-peptide comprend un affimer.

3. Système selon la revendication 1, dans lequel le premier agent donneur comprend un premier anticorps.

4. Système selon la revendication 3, dans lequel le deuxième agent accepteur est un deuxième agent accepteur marqué comprenant une pluralité de deuxièmes marqueurs peptidiques liés de manière covalente.

5. Système selon l'une quelconque des revendications 1 à 4, dans lequel la bille donneuse est revêtue de streptavidine ou de l'un de ses dérivés.

6. Système selon la revendication 1, dans lequel le premier agent accepteur marqué est un deuxième anticorps lié de manière covalente à au moins un marqueur peptidique ou à une pluralité de marqueurs peptidiques pour former un deuxième anticorps marqué.

7. Système selon la revendication 6, dans lequel le deuxième anticorps marqué comprend un épitope d'un anticorps anti-peptide attaché, conjugué ou lié de manière covalente à une première bille acceptrice.

8. Système selon la revendication 1, dans lequel la bille donneuse est dopée avec un photosensibilisateur capable d'émettre de l'oxygène singulet lorsqu'elle est stimulée par la lumière à une longueur d'onde dans la plage allant de 250 à 1100 nm, de 300 à 1000 nm, de 650 à 700 nm ou de 660 à 680 nm.

9. Système selon la revendication 1, dans lequel la première bille acceptrice comprend un indicateur photoactif qui réagit avec l'oxygène singulet formant un indicateur photoactif capable de devenir fluorescent, où l'irradiation de l'indicateur photoactif permet d'émettre une fluorescence à une longueur d'onde dans la plage allant de 500 à 625 nm, de 525 à 575 nm, de 525 à 550 nm ou de 540 à 560 nm.

10. Système selon la revendication 1, dans lequel la bille donneuse et la première bille acceptrice ont chacune un diamètre dans la plage allant de 50 à 500 nm, de 100 à 500 nm, de 150 à 350 nm, de 250 à 350 nm ou de 250 à 275 nm.


### SEQUENCE LISTING

<110> TGR BIOSCIENCES PTY LTD.
<120> ANALYTE DETECTION WITH MULTIPLE SUBSTRATES
<130> 013018-0008-228
<140>
   <141>
<150> 62/237,522
   <151> 2015-10-05
<150> 62/236,676
   <151> 2015-10-02
<160> 8
<170> PatentIn version 3.5
<210> 1
   <211> 8
   <212> PRT
   <213> Artificial Sequence
<220>
   <223> Description of Artificial Sequence: Synthetic peptide
<400> 1
   Asp Tyr Lys Asp Asp Asp Asp Lys
   1               5
<210> 2
   <211> 23
   <212> PRT
   <213> Artificial Sequence
<220>
   <223> Description of Artificial Sequence: Synthetic peptide
<400> 2
   Lys Arg Ile Thr Val Glu Glu Ala Leu Ala His Pro Tyr Leu Glu Gln
   1               5                   10                  15
   Tyr Tyr Asp Pro Thr Asp Glu
               20
<210> 3
   <211> 6
   <212> PRT
   <213> Artificial Sequence
<220>
   <223> Description of Artificial Sequence: Synthetic 6xHis tag
<400> 3
   His His His His His His
   1 5
<210> 4
   <211> 10
   <212> PRT
   <213> Artificial Sequence
<220>
   <223> Description of Artificial Sequence: Synthetic peptide
<400> 4
   Glu Gln Lys Leu Ile Ser Glu Glu Asp Leu
   1 5 10
<210> 5
   <211> 9
   <212> PRT
   <213> Artificial Sequence
<220>
   <223> Description of Artificial Sequence: Synthetic peptide
<400> 5
   Tyr Pro Tyr Asp Val Pro Asp Tyr Ala
   1 5
<210> 6
   <211> 11
   <212> PRT
   <213> Artificial Sequence
<220>
   <223> Description of Artificial Sequence: Synthetic peptide
<400> 6
   Tyr Thr Asp Ile Glu Met Asn Arg Leu Gly Lys
   1 5 10
<210> 7
   <211> 11
   <212> PRT
   <213> Artificial Sequence
<220>
   <223> Description of Artificial Sequence: Synthetic peptide
<400> 7
   Gln Pro Glu Leu Ala Pro Glu Asp Pro Glu Asp
   1 5 10
<210> 8
   <211> 9
   <212> PRT
   <213> Artificial Sequence
<220>
   <223> Description of Artificial Sequence: Synthetic peptide
<400> 8
   Cys Asp Tyr Lys Asp Asp Asp Asp Lys
   1 5